Europäisches Patentamt

(19) European Patent Office

Office européen des brevets

(11) Publication number: **0 007 691**
**B1**

(12) **EUROPEAN PATENT SPECIFICATION**

(45) Date of publication of patent specification: **18.01.84**

(21) Application number: **79301048.9**

(22) Date of filing: **04.06.79**

(51) Int. Cl.³: **A 61 K 31/70** //(A61K31/70, 31/405, 31/195)

(54) Compositions for use in decreasing appetite for calories as carbohydrates.

(30) Priority: **31.07.78 US 929387**

(43) Date of publication of application:
**06.02.80 Bulletin 80/3**

(45) Publication of the grant of the patent:
**18.01.84 Bulletin 84/3**

(84) Designated Contracting States:
**AT BE CH DE FR GB IT LU NL SE**

(56) References cited:
EP - A - 0 005 957
FR - A - 2 181 462
FR - A - 2 305 977
GB - A - 949 665
GB - A - 1 289 096

UNLISTED DRUGS, vol. 23, August 1971,
Chatham, N.J., U.S.A. "OPTIMAX" p.116m
DICTIONNAIRE VIDAL, 1967 O.V.P., Paris
"ENDAMINE" page 480 "HYDROLYSAT DE
PROTEINES LABAZ" page 681
MODERN DRUG ENCYCLOPEDIA, 1961, eighth
edition, THE REUBEN H. DONNELLEY
CORPORATION, New York "PROTINAL
POWDER" page 1038

(73) Proprietor: **MASSACHUSETTS INSTITUTE OF TECHNOLOGY**
**Room E19-722 77 Massachusetts Avenue Cambridge, MA 02139 (US)**

(72) Inventor: **Wurtman, Richard J.**
**271 Woodward Street**
**Waban Massachusetts (US)**
Inventor: **Fernstrom, John D.**
**65 East India Row**
**Boston Massachusetts (US)**
Inventor: **Wurtman, Judith J.**
**271 Woodward Street**
**Waban Massachusetts (US)**

(74) Representative: **Holdcroft, James Gerald, Dr. et al,**
**Graham Watt & Co. Riverhead**
**Sevenoaks Kent TN13 2BN (GB)**

(56) References cited:
The Journal of Physical Chemistry, Vol. 80,
No.3, 1976, pages 249-252 T.S. LAKSHML et
al.: "Effects of Sugar Solutions on the Activity
Coefficients of Aromatic Amino Acids and their
N-Acetyl-Ethyl Esters"

Courier Press, Leamington Spa, England.

Compositions for use in decreasing appetite for calories as carbohydrates

This invention relates to compositions for suppressing calorie consumption by animals.

Presently, appetite is suppressed in order to control intake of calories by administering certain drugs or bulking substances. Commonly, drugs like dexedrine or d-amphetamine have been administered orally and have been found effective in suppressing appetite. However, each of these drugs has unwanted side effects (such as induction of hyperactivity or even psychosis) and in some instances can be dangerous to the user. In addition, the amphetamines do not exhibit selectivity in suppressing calorie-carbohydrate intake as they also cause a suppression in protein consumption. This feature makes them disadvantageous, especially in obese adolescents. Similarly, the use of carbohydrates or bulking sustances have been found to be relatively ineffective in suppressing appetite.

It is one object of the present invention to provide for suppressing appetite in a selective manner such that the appetite for high caloric foods is suppressed while at the same time appetite for protein substances is less affected.

It is another object of the invention to minimize unwanted side effects in the suppression of appetite e.g. those that occur with drugs such as the amphetamines.

We have developed a composition which we believe suppresses appetite for calories (as carbohydrates) while elevating the percent of total calories that is consumed as protein. We have found that a combination of tryptophan and a carbohydrate which causes insulin secretion selectively suppresses the appetite for calories (as carbohydrates). A mixture of tryptophan and an insulin-releasing carbohydrate can be administered alone, in admixture with tyrosine, or with caffeine or another mild stimulant, to override the mixture's natural sedating effects. It is believed that these compositions function by mechanisms which involve the enhancement of brain serotonin synthesis; this neurotransmitter is involved in the control of appetite. Tyrosine, is a precursor for dopamine and norepinephrine in synapses. Thus, a composition which also includes tyrosine permits serotonin synthesis to be accelerated while not reducing the synthesis of dopamine and/or norepinephrine in synapses; in some situations, such as when it is necessary to be fully attentive, it is important not to lower dopamine and/or norepinephrine synthesis.

The invention provides a composition which, when administered, decreases appetite for calories as carbohydrates characterised in that the composition comprises (a) an insulin releasing carbohydrate and (b), as the only neutral amino acid(s) of the kind normally found in blood plasma, tryptophan and optionally tyrosine, and the composition is substantially free of other components which inhibit tryptophan uptake into the brain; and the weight ratio of carbohydrate to tryptophan is between 3 and 50.

Said ratio is for example between 3 and 30; between 3 and 20; or between 3 and 5.

The compositions may be used as sweeteners.

The compositions may also comprise an excipient.

Tryptophan-containing compositions are disclosed in the following prior documents: "Unlisted Drugs" Vol. 23 August 1971, Chatam N. J. U.S.A.—"Optimax" p. 116 m; French Patent Application A—2,181,462 (Johnson and Johnson); U.K. Patent Specification A—1,289,096 (Cocheme *et al*); "Dictionnaire Vidal", 1967 OVP, Paris, "Endamine" p. 48, "Hydrolysat de Proteines Labaz" p. 681.

However the objectives of these prior disclosures are different from those of the present invention and indeed the prior compositions would not achieve the effects of the invention.

The "Unlisted Drugs" reference and U.K. Specification are concerned with the treatment of depressive illnesses including for example loss of appetite. The French Patent Application is concerned with dietary compositions for use when normal digestive activity is reduced. The "Dictionnaire Vidal" reference is concerned with nutritional solutions for injection e.g. drip-feeds and again the objective is the provision of a balanced dietary composition.

In the "Unlisted Drugs" reference the ratio of carbohydrate to tryptophan is 2 i.e. below the lower limit of the present invention, and this would not provide sufficient insulin release to obtain the effects of the present invention. This applies similarly to Examples 1, 2 and 2(b) of the U.K. Specification however Example 3 of the U.K. Specification does disclose a ratio of D-sorbitol to tryptophan of 10, but D-sorbitol is not effective to release insulin from the pancreas. Also all the compositions of "Unlisted Drugs" and the U.K. Specification contain pyridoxine which would increase liver function to decrease tryptophan and reduce availability of tryptophan to the brain.

The specific compositions of the French Patent Application all contain amino acids which would compete with tryptophan for uptake into the brain thereby inhibiting tryptophan uptake; and the French Application does not disclose compositions which are free of components inhibiting tryptophan uptake into the brain. Again, the ratios of carbohydrate to tryptophan in the French Application are well above the upper limit of 50 of the present invention; at the high figures of the French Application there would be quite insufficient tryptophan to be effective for the purposes of the present invention.

Both the compositions of the "Dictionnaire Vidal" reference include a protein hydrolysate which would contain competing amino acids inhibiting tryptophan uptake into the brain. Furthermore there is far too little tryptophan present in the compositions of this reference (only trace amounts).

There now follows a description of embodiments of the invention. This description is given by way of example only, and not by way of limitation of the invention.

In, for example, treating an obese patient utilising the present invention, tryptophan and an insulin-releasing carbohydrate are administered to the patient. When there is need to sustain or increase brain dopamine or norepinephrine levels, the compositions embodying the invention also contain tyrosine in addition to the tryptophan and the carbohydrate that causes insulin to be released. The administration of tryptophan changes the ratio of tryptophan to the sum of the plasma concentrations of other neutral amino acids that compete with tryptophan for uptake in the brain thereby increasing the brain serotonin level. Furthermore, the administration of a carbohydrate that releases insulin decreases the plasma levels of the other neutral amino acids normally found in the plasma such as leucine, isoleucine, tyrosine, phenylalanine and valine. Thus, the carbohydrate causes an increase of the plasma levels of tryptophan in relation to these other amino acids by decreasing the concentration of the other amino acids in the plasma. Both of these effects are cumulative in effecting an increase in brain serotonin levels. While we do not intend to be bound by theory, it is believed that the selective suppression of appetite for calories is caused by such increases in brain serotonin levels.

Representative suitable carbohydrates include sucrose, dextrose, starch, fructose, invert sugar, dextrins, sugar polymers such as polyose, xylitol and mixtures thereof. We believe the relative proportion of tryptophan to the insulin-releasing carbohydrate can vary widely so long as there is a cumulative effect on brain serotonin levels by the two components utilized; and the weight ratio of the carbohydrate to tryptophan is between 3 and 50 generally up to 20 and more usually up to 5. The compositions embodying this invention are administered in amounts sufficient to effect increase in brain serotonin levels while not being administered in such large amounts as to seriously reduce the brain levels of other neurotransmitters needed for normal functioning such as dopamine, norepinephrine, acetylocholine, or the non-essential amino acids. Generally, the compositions are administered in an amount of between 10 mg/kg and 100 mg/kg of tryptophan, and up to 300 mg/kg of carbohydrate, more usually between 20 mg/kg and 50 mg/kg of tryptophan, and up to 150 mg/kg of carbohydrate. Typical unit dosage form useful for oral administration ranges between 0.5 grams and 15 grams, and more usually between 1 gram and 10 grams.

The tryptophan and tyrosine can be administered as free amino acids, esters, salts neutral or synthetic polymers. The route of administration will generally be oral, for example, as a tablet, sustained-release capsule, drink, beverage sweetener, wafer, candy, chewing gum. It may be mixed with a mild stimulant like caffeine for daytime use (to override the sedative effect of tryptophan), or used *without* a mild stimulant at night (for example, by people with nocturnal eating problems).

Example

This Example illustrates that the administration of a diet containing an insulin-releasing carbohydrate and tryptophan effects a reduction in appetite for carbohydrates.

Male Sprague-Dally rats (Charles River Breeding Laboratory, Wilmington Massachusetts) weighing 130 grams were housed in cages (1 per cage), and given *ad libitum* access to tap water and, for 8 hours each day, two test diets comprising protein (casein), corn oil, carbohydrate (dextrin), vitamins and minerals. One of the test diets contained 5% protein and the other test diet contained 45% protein. The rats were maintained under light (300 microwatts/cm²); Vita-Lite, Duro-Test Corp., Northburg, New Jersey, between 8:00 a.m. and 8:00 p.m. daily. The control values obtained in the Table below are from data obtained on the same rats as used during the actual test on the day preceding administration of tryptophan and sucrose.

After the control data were obtained, each of the rats was given a 3 gram mixture containing 1.5 grams sucrose, 30 mg tryptophan and 1.5 ml water. This diet was consumed within 15 minutes and 30 minutes thereafter each of the rats was given access to the two test diets containing respectively 5% and 45% protein. Food consumption by each rat was measured 45 minutes after the animals had been given access to the test diets. The results are shown in the Table; each of the rats given the three meal diet containing tryptophan and sucrose consumed approximately 50% less carbohydrate food on the average as compared to the control data.

TABLE

| Group | Food intake | %-Protein ingested |
|---|---|---|
| | (grams) | |
| Control | 10.5±1.0 | 25.0±1.7 |
| Pre-meal | 5.6±1.0* | 31.0±1.8** |

*$p<0.001$
**$p<0.02$

**Claims for the Contracting States: BE CH DE FR GB IT LU NL SE**

1. A composition which, when administered, decreases appetite for calories as carbohydrates characterised in that the composition comprises (a) an insulin releasing carbohydrate and (b), as the only neutral amino acid(s) of the kind normally found in blood plasma, tryptophan and optionally tyrosine, and the composition is substantially free of other components which inhibit tryptophan uptake into the brain; and the weight ratio of carbohydrate to tryptophan is between 3 and 50.

2. A composition according to claim 1, wherein said ratio is between 3 and 30.

3. A composition according to claim 1, wherein said ratio is between 3 and 20.

4. A composition according to claim 1, wherein said ratio is between 3 and 5.

5. A composition according to any one of the preceding claims in unit dosage form.

6. A composition according to any one of the preceding claims, wherein the carbohydrate is a sugar.

7. A composition according to claim 6, wherein the sugar is sucrose.

8. A composition according to any one of the preceding claims which also includes a mild stimulant.

9. A composition according to claim 8, wherein the stimulant is caffeine.

10. A composition according to claim 1, wherein the components of the composition are present in admixture.

11. A composition according to any one of the preceding claims suitable for administration in a dosage of between 10 mg/kg and 100 mg/kg tryptophan and up to 300 mg/kg carbohydrate.

12. A composition according to claim 11, wherein said dosage is between 20 mg/kg and 50 mg/kg tryptophan and up to 150 mg/kg carbohydrate.

**Claims for the Contracting State: AT**

1. A method of manufacturing a composition which, when administered, decreases appetite for calories as carbohydrates characterised by mixing (a) an insulin-releasing carbohydrate with (b), as the only neutral amino acid(s) present, of the kind normally found in the blood plasma, tryptophan and optionally tyrosine; while maintaining said composition substantially free of other components which inhibit tryptophan uptake into the brain; and in that the weight ratio of carbohydrate to tryptophan is between 3 and 50.

2. A method according to claim 1, wherein said ratio is between 3 and 30.

3. A method according to claim 1, wherein said ratio is between 3 and 20.

4. A method according to claim 1, wherein said ratio is between 3 and 5.

5. A method according to any one of the preceding claims, wherein the composition is manufactured in unit dosage form.

6. A method according to any one of the preceding claims, wherein the carbohydrate is a sugar.

7. A method according to claim 6, wherein the sugar is sucrose.

8. A method according to any one of the preceding claims in which a mild stimulant is also included in the composition.

9. A method according to claim 8, wherein the stimulant is caffeine.

10. A method according to any one of the preceding claims, wherein the composition is manufactured suitable for administration in a dosage of between 10 mg/kg and 100 mg/kg tryptophan and up to 300 mg/kg carbohydrate.

11. A method according to claim 10, wherein said dosage is between 20 mg/kg and 50 mg/kg tryptophan and up to 150 mg/kg carbohydrate.

**Patentansprüche für die Vertragsstaaten: BE CH DE FR GB IT LU NL SE**

1. Mittel zur Verabreichung zwecks Verminderung des Appetits auf Kohlenhydrat-Kalorien, dadurch gekennzeichnet, dass es (a) ein Insulinausschüttung verursachendes Kohlenhydrat und (b) als einzige neutrale, normalerweise in Blutplasma vorkommende Aminosäuren Tryptophan und gegebenenfalls Tyrosin enthält, dass es praktisch frei von Bestandteilen ist, welche eine Tryptophanaufnahme durch das Gehirn inhibieren, und dass das Gewichsverhältnis von Kohlenhydrat zu Tryptophan zwischen 3 und 50 beträgt.

2. Mittel nach Anspruch 1, dadurch gekennzeichnet, dass das genannte Verhältnis 3 bis 30 beträgt.

3. Mittel nach Anspruch 1, dadurch gekennzeichnet, dass das genannte Verhältnis 3 bis 20 beträgt.

4. Mittel nach Anspruch 1, dadurch gekennzeichnet, dass das genannte Verhältnis 3 bis 5 beträgt.

5. Mittel nach einem der vorstehenden Ansprüche in Form von Einheitsdosen.

6. Mittel nach einem der vorstehenden Ansprüche, dadurch gekennzeichnet, dass das Kohlenhydrat ein Zucker ist.

7. Mittel nach Anspruch 6, dadurch gekennzeichnet, dass der Zucker Sucrose ist.

8. Mittel nach einem der vorstehenden Ansprüche, welches weiterhin ein schwaches Stimulans enthält.

9. Mittel nach Anspruch 8, dadurch gekennzeichnet, dass das Stimulans Koffein ist.

10. Mittel nach Anspruch 1, dadurch gekennzeichnet, dass seine Komponenten in Mischung miteinander vorliegen.

11. Mittel nach einem der vorstehenden Ansprüche, geeignet zur Verabreichung einer Dosierung entsprechend 10 mg/kg bis 100 mg/kg Tryptophan und bis zu 300 mg/kg Kohlenhydrat.

12. Mittel nach Anspruch 11, dadurch gekennzeichnet, dass die Dosierung 20 mg/kg bis 50 mg/kg Tryptophan und bis zu 150 mg/kg Kohlenhydrat entspricht.

### Patentansprüche für den Vertragsstaat AT

1. Verfahren zur Herstellung eines Mittels, welches bei Verabreichung den Appetit auf Kohlenhydrat-Kalorien vermindert, dadurch gekennzeichnet, dass man (a) ein Insulinausschüttung verursachendes Kohlenhydrat mit (b) als einzigen neutralen, normalerweise in Blutplasma vorkommenden Aminosäuren Tryptophan und gegebenenfalls Tyrosin vermischt, wobei man das Mittel praktisch frei von anderen Bestandteilen hält, welche die Tryptophanaufnahme durch das Gehirn inhibieren, und wobei das Gewichtsverhältnis von Kohlenhydrat zu Tryptophan zwischen 3 und 50 beträgt.

2. Verfahren nach Anspruch 1, dadurch gekennzeichnet, dass das genannte Verhältnis 3 bis 30 beträgt.

3. Verfahren nach Anspruch 1, dadurch gekennzeichnet, dass das genannte Verhältnis 3 bis 20 beträgt.

4. Verfahren nach Anspruch 1, dadurch gekennzeichnet, dass das genannte Verhältnis 3 bis 5 beträgt.

5. Verfahren nach einem der vorstehenden Ansprüche, dadurch gekennzeichnet, dass man das Mittel in Einheitsdosen herstellt.

6. Verfahren nach einem der vorstehenden Ansprüche, dadurch gekennzeichnet, dass das Kohlenhydrat ein Zucker ist.

7. Verfahren nach Anspruch 6, dadurch gekennzeichnet, dass der Zucker Sucrose ist.

8. Verfahren nach einem der vorstehenden Ansprüche, dadurch gekennzeichnet, dass man dem Mittel weiterhin ein schwaches Stimulans einverleibt.

9. Verfahren nach Anspruch 8, dadurch gekennzeichnet, dass das Stimulans Koffein ist.

10. Verfahren nach einem der vorstehenden Ansprüche, dadurch gekennzeichnet, dass man das Mittel für eine Verabreichung mit einer Dosierung entsprechend 10 mg/kg bis 100 mg/kg Tryptophan und bis zu 300 mg/kg Kohlenhydrat herstellt.

11. Verfahren nach Anspruch 10, dadurch gekennzeichnet, dass die Dosierung 20 mg/kg bis 50 mg/kg Tryptophan und bis zu 150 mg/kg Kohlenhydrat entspricht.

### Revendications pour les États Contractants: BE CH DE FR GB IT LU NL SE

1. Composition qui, après l'administration, diminue l'appétit pour des calories sous forme d'hydrates de carbone, caractérisée en ce qu'elle comprend (a) un hydrate de carbone libérant de l'insuline, et (b) du tryptophane et, éventuellement, de la tyrosine comme seuls acides aminés neutres du genre normalement présent dans le plasma sanguin, et que la composition est substantiellement exempte d'autres composants inhibant l'absorption de tryptophane par le cerveau; le rapport pondéral d'hydrates de carbone au tryptophane étant compris entre 3 et 50.

2. Composition selon la revendication 1, caractérisée en ce que le dit rapport est compris entre 3 et 30.

3. Composition selon la revendication 1, caractérisée en ce que le dit rapport est compris entre 3 et 20.

4. Composition selon la revendication 1, caractérisée en ce que le dit rapport est compris entre 3 et 5.

5. Composition selon l'une quelconque des revendications précédentes sous forme de doses standardisées.

6. Composition selon l'une quelconque des revendications précédentes où l'hydrate de carbone est un sucre.

7. Composition selon la revendication 6 où le sucre est le sucrose.

8. Composition selon l'une quelconque des revendications précédentes qui comprend également un léger stimulant.

9. Composition selon la revendication 8 où le stimulant est la caféine.

10. Composition selon la revendication 1 comprenant composants en mélange.

11. Composition selon l'une quelconque des revendications précédentes, appropriée pour l'administration à un dosage compris entre 10 mg/kg et 100 mg/kg de tryptophane et jusqu'à 300 mg/kg d'hydrate de carbone.

12. Composition selon la revendication 11 où le dit dosage est compris entre 20 mg/kg et 50 mg/kg de tryptophane et jusqu'à 150 mg/kg d'hydrate de carbone.

## Revendications pour l'État Contractant AT

1. Procédé de préparation d'une composition qui, après administration, diminue l'appétit pour des calories sous forme d'hydrates de carbone, caractérisé en ce qu'on mélange (a) un hydrate de carbone libérant de l'insuline avec (b) du tryptophane et, éventuellement, de la tyrosine comme seuls acides aminés neutres du genre normalement présent dans le plasma sanguin, tout en maintenant la composition substantiellement exempte d'autres composants inhibant l'absorption de tryptophane par le cerveau; le rapport pondéral d'hydrate de carbone au tryptophane étant choisi entre 3 et 50.

2. Procédé selon la revendication 1, caractérisé en ce que le dit rapport est compris entre 3 et 30.

3. Procédé selon la revendication 1, caractérisé en ce que le dit rapport est compris entre 3 et 20.

4. Procédé selon la revendication 1, caractérisé en ce que le dit rapport est compris entre 3 et 5.

5. Procédé selon l'un quelconque des revendications précédentes, caractérisé en ce que la composition est préparée sous forme de doses standardisées.

6. Procédé selon l'une quelconque des revendications précédentes, caractérisé en ce que l'hydrate de carbone est un sucre.

7. Procédé selon la revendication 6, caractérisé en ce que le sucre est le sucrose.

8. Procédé selon l'une quelconque des revendications précédentes, caractérisé en ce qu'on incorpore également un léger stimulant dans la composition.

9. Procédé selon la revendication 8, caractérisé en ce que le stimulant est la caféine.

10. Procédé selon l'une quelconque des revendications précédentes, caractérisé en ce que la composition est préparée sous forme appropriée pour l'administration sous forme de doses comprenant entre 10 mg/kg et 100 mg/kg de tryptophane et jusqu'à 300 mg/kg d'hydrates de carbone.

11. Procédé selon la revendication 10, caractérisé en ce que le dit dosage est compris entre 20 mg/kg et 50 mg/kg de tryptophane et jusqu'à 150 mg/kg d'hydrates de carbone.